(19) Europäisches Patentamt
European Patent Office
Office européen des brevets

(11) EP 0 895 775 B1

(12) EUROPÄISCHE PATENTSCHRIFT

(45) Veröffentlichungstag und Bekanntmachung des Hinweises auf die Patenterteilung:
**30.06.2004 Patentblatt 2004/27**

(51) Int Cl.[7]: **A61K 7/42**

(21) Anmeldenummer: **98113035.4**

(22) Anmeldetag: **14.07.1998**

(54) **Lichtschutzbereitungen mit einem Gehalt an anorganischen Pigmenten, welche oberflächlich mit UV-Filtersubstanzen beschichtet sind**

Lightprotection compositions containing inorganic pigments which are coated on their surface with UV filter substances

Compositions photoprotectives contenants des pigments inorganiques enrobées sur leurs surfaces avec des filtres UV

(84) Benannte Vertragsstaaten:
**AT BE CH DE ES FR GB IT LI NL SE**

(30) Priorität: **31.07.1997 DE 19732954**

(43) Veröffentlichungstag der Anmeldung:
**10.02.1999 Patentblatt 1999/06**

(73) Patentinhaber: **Beiersdorf Aktiengesellschaft
20245 Hamburg (DE)**

(72) Erfinder:
- **Gers-Barlag, Heinrich Dr.
25495 Kummerfeld (DE)**
- **Rupprecht, Herbert Prof. Dr.
93053 Regensburg (DE)**
- **Vogl, Sabine Dr.
40723 Hilden (DE)**

(56) Entgegenhaltungen:
**EP-A1- 0 587 908**
**EP-A1- 0 748 624**
**WO-A1-90/06974**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 0 895 775 B1

**Beschreibung**

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Bekannte und vorteilhafte, unter Normalbedingungen als Festkörper vorliegende Lichtschutzfiltersubstanz sind Dibenzoylmethanderivate, beispielsweise 5-Isopropyldibenzoylmethan (CAS-Nr. 63250-25-9), welches sich durch die Struktur

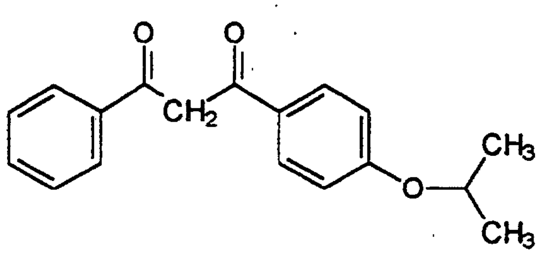

auszeichnet und von Merck unter der Marke Eusolex® 8020 verkauft wird, sowie das 4-(tert.-Butyl)-4'-methoxydibenzoylmethan (CAS-Nr. 70356-09-1), welches sich durch die Struktur

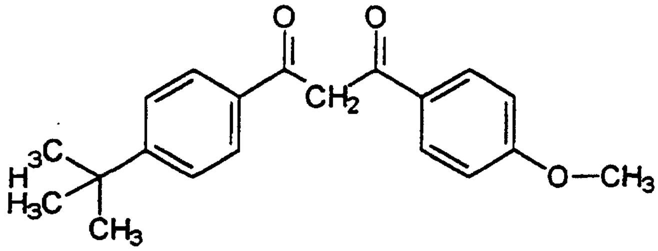

auszeichnet, und von Givaudan unter der Marke Parsol® 1789 verkauft wird.

Der Hauptnachteil von Dibenzoylmethanderivaten ist eine gewisse Instabilität gegenüber UV-Strahlung, so daß es zweckmäßig ist, Zubereitungen mit einem Gehalt an dieser Substanz auch gewisse UV-Stabilisatoren einzuverleiben. Die photochemische Zersetzung von 4-(tert.-Butyl)-4'-methoxydibenzoylmethan - stellvertretend für alle im UV-Bereich absorbierenden Dibenzoylmethanderivate - folgt einer Norrish-Typ-I-Acylspaltung gemäß dem nachfolgenden Reaktionsschema:

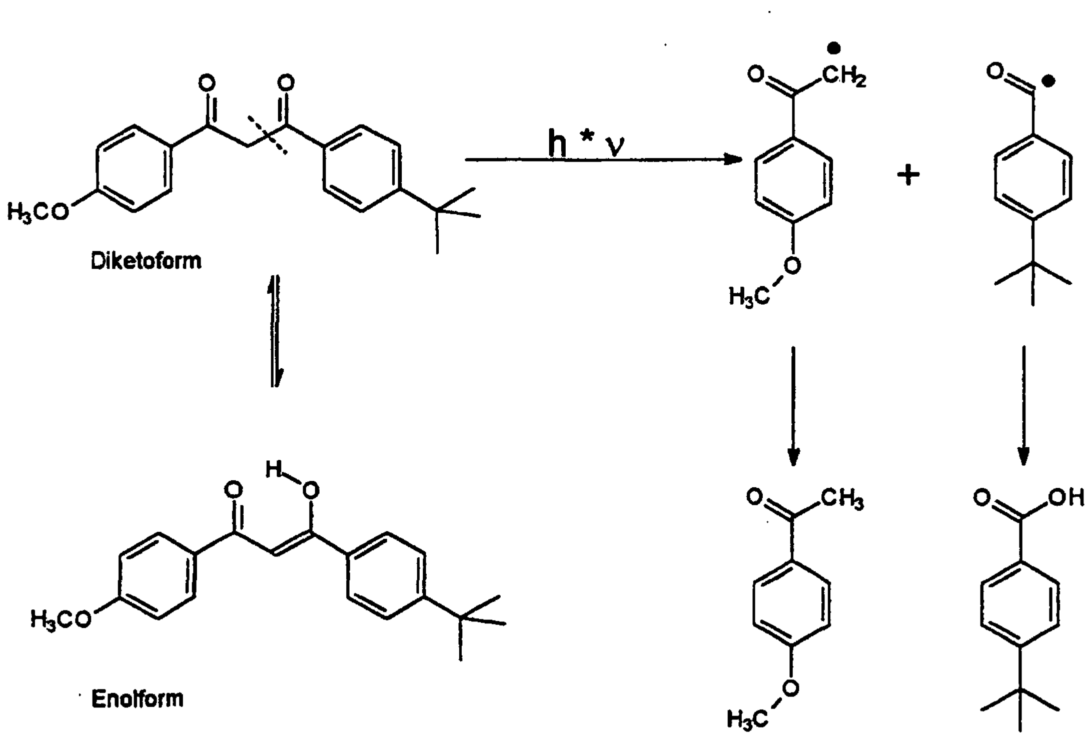


Die Reaktionsprodukte stehen als Lichtschutzfiltersubstanzen nicht mehr zur Verfügung. Es bestand daher ein dringender Bedarf, Wege aufzuweisen, auf welchen der photolytischen Zersetzung von Dibenzoylmethanderivaten wirksam begegnet werden kann.

Beispielsweise beschreibt die deutsche Offenlegungsschrift DE-A-37 41 420 die Kombination dieses Lichtschutzfilters in bestimmtem Mengenverhältnis zu 4-Methylbenzylidencampher, welcher sich durch die Struktur

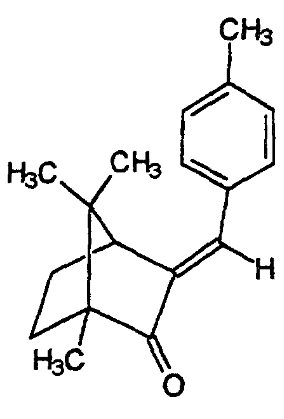

auszeichnet und von Merck unter der Marke Eusolex® 6300 verkauft wird. Die a.a.O. beschriebenen Zubereitungen zeichnen sich aber wiederum durch andere Nachteile aus, hauptsächlich formulierungstechnischer Natur.

4-Methylbenzylidencampher ist eine äußerst vorteilhafte, unter Normalbedingungen als Festkörper vorliegende Lichtschutzfiltersubstanz und zeichnet sich an sich durch gute UV-Filtereigenschaften aus. Gerade in Kombination mit anderen als Festkörper vorliegenden Substanzen ist ihre Einsatzkonzentration jedoch begrenzt.

Auch andere Benzylidencampherderivate liegen unter Normalbedingungen als Festkörper vor und sind vorteilhafte Lichtschutzfiltersubstanzen, z.B. der Benzylidencampher, welcher sich durch die Struktur

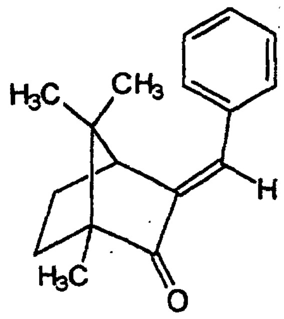

auszeichnet und von der Gesellschaft Induchem unter der Marke Unisol® S22 verkauft wird.

Ein weiterer vorteilhafter, unter Normalbedingungen als Festkörper vorliegender UVB-Filter ist der 4,4', 4''-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin.

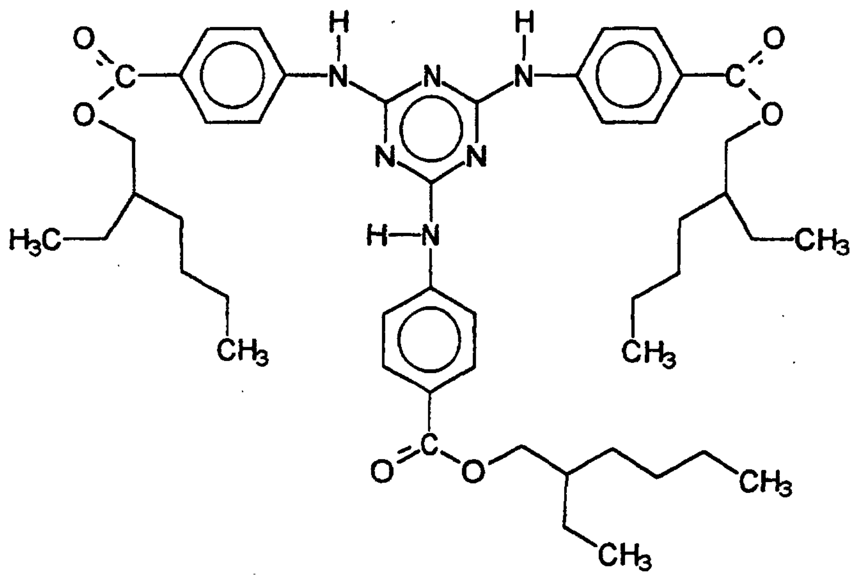

Diese UVB-Filtersubstanz wird von der BASF Aktiengesellschaft unter der Warenbezeichnung UVINUL® T 150 vertrieben und zeichnet sich durch gute UV-Absorptionseigenschaften aus. Zwischenzeitlich wurden von verschiedenen Autoren andere UV-Filtersubstanzen beschrieben, welche unter Normalbedingungen als Festkörper vorliegen und das Strukturmotiv

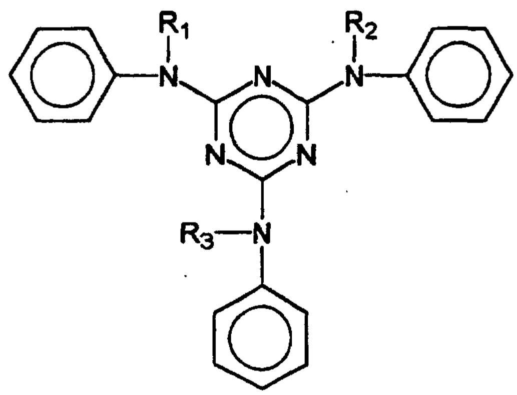

aufweisen.

Der Hauptnachteil des 4,4',4"-(1,3,5-Triazin-2,4,6-triyltriimino)-tris-benzoesäuretris(2-ethylhexylesters) ist seine schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Fifter-substanz.

Weitere unter Normalbedingungen als Festkörper vorliegende UV-Fiftersubstanzen, zeichnen sich durch das Strukturmotiv des Benzophenons

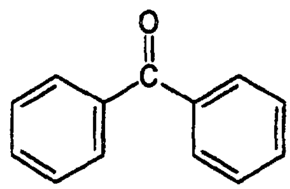

aus, beispielsweise gewählt aus der Gruppe der folgenden Substanzen

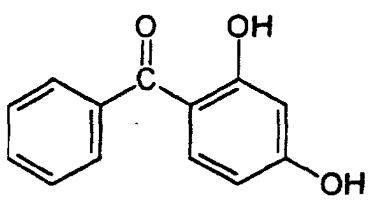

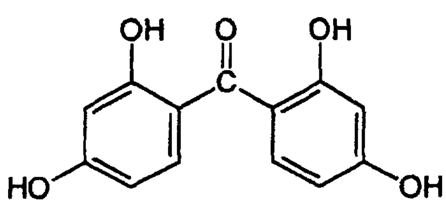

**Benzophenon-1**
**(2,4-Dihydroxybenzophenon)**

**CAS-Nr. 131-56-6**

**Benzophenon-2**
**(2,2',4,4'-Tetrahydroxybenzophenon)**
**CAS-Nr. 131-55-5**

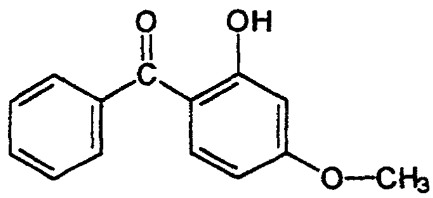

Benzophenon-3
(2-Hydroxy-4-methoxybenzophenon)

CAS-Nr. 131-57-7

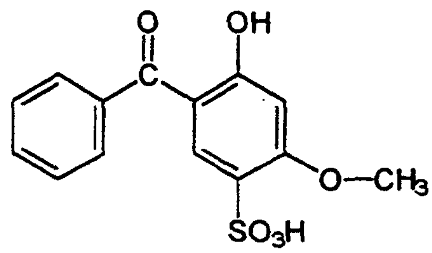

Benzophenon-4
(2-Hydroxy-4-methoxybenzophenon-5-sulfonsäure)
CAS-Nr. 4065-45-6

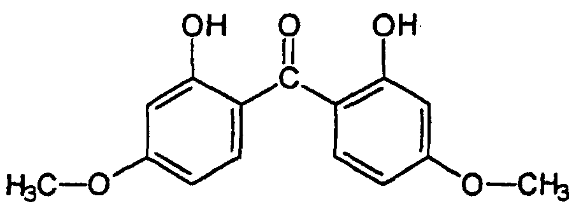

Benzophenon-6
(2,2'-Dihydroxy-4,4'-dimethoxybenzophenon)

CAS-Nr. 131-54-4

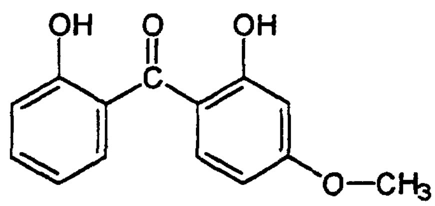

Benzophenon-8
(2,2'-Dihydroxy-4-methoxybenzophenon)
CAS-Nr. 131-53-3

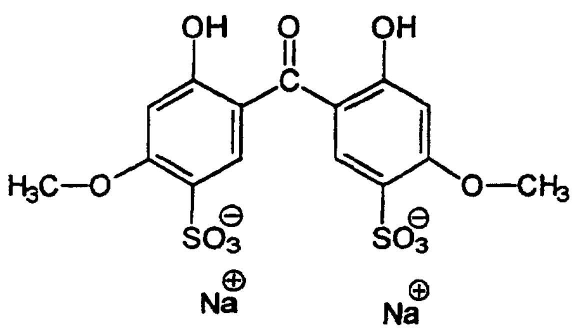

Benzophenon-9
(Dinatrium-2,2'-dihydroxy-4,4'-dimethoxy-5,5'-disulfobenzophenon)
CAS-Nr. 3121-60-6,

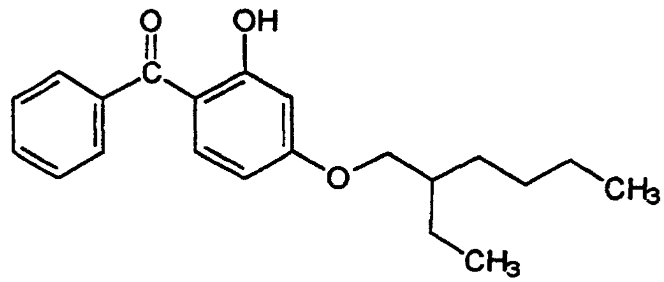

**Benzophenon-12,**
**(2-Hydroxy-4-(2-ethylhexyloxy)-benzophenon)**
**CAS-Nr. 1843-05-6,**

**ferner Benzophenon-11, CAS-Nr. 1341-54-4.**

Ferner existieren unter der allgemeinen Strukturformel der 2-cyano-3,3'-diphenylacrylsäurealkylester

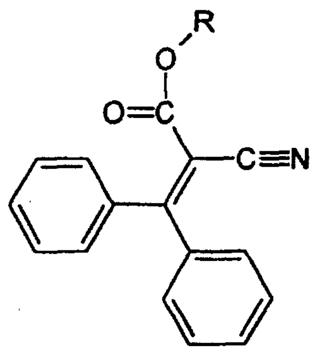

einige Vertreter, die unter Normalbedingungen als Festkörper vorliegen, beispielsweise das 2-Ethyl-2-cyan-3,3'-diphenylacrylat (Etocrylen), welches die Strukturformel

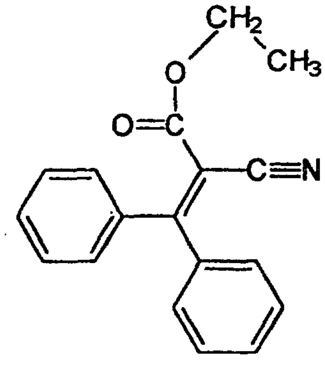

**CAS-Nr. 5232-99-5**

besitzt und von der BASF unter der Bezeichnung UVINUL N-35 verkauft wird.

Weitere vorteilhafte unter Normalbedingungen als Festkörper vorliegende Lichtschutzsubstanzen sind wasserlöslich und tragen in der Regel eine Sulfonatgruppe wie z.B. die 2-Phenylbenzimidazol-5-sulfonsäure und ihre Salze, beispielsweise das Natrium-, Kalium- oder ihr Triethanolammonium-Salz

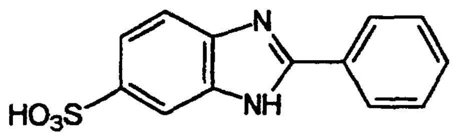

,

Sulfonsäure-Derivate von Benzophenonen, vorzugsweise 2-Hydroxy-4-methoxybenzophenon-5-sulfonsäure und ihre Salze, beispielsweise das entsprechende Natrium-, Kalium- oder Triethanolammonium-Salz:

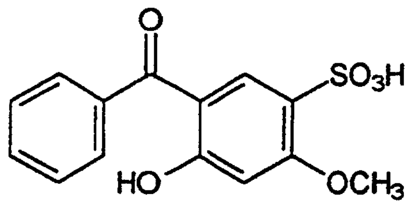

Sulfonsäure-Derivate des 3-Benzylidencamphers, wie z.B. 4-(2-Oxo-3-bornylidenmethyl)benzolsulfonsäure und ihre Salze, beispielsweise das entsprechende Natrium-, Kalium- oder Triethanolammonium-Salz:

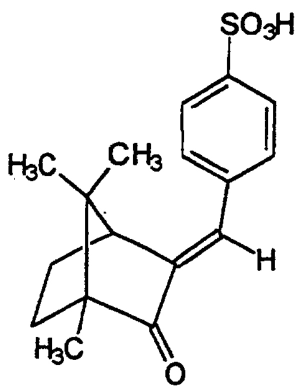

Die 2-Methyl-5-(2-oxo-3-bornylidenmethyl)benzolsulfonsäure und ihre Salze, beispielsweise das entsprechende Natrium-, Kalium- oder Triethanolammonium-Salz:

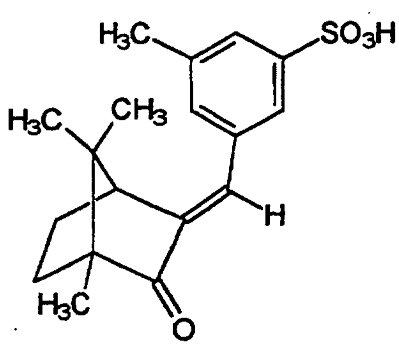

Das 1,4-di(2-oxo-10-Sulfo-3-bomylidenmethyl-)Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bomylidenmethyl-10-Sulfonsäure bezeichnet:

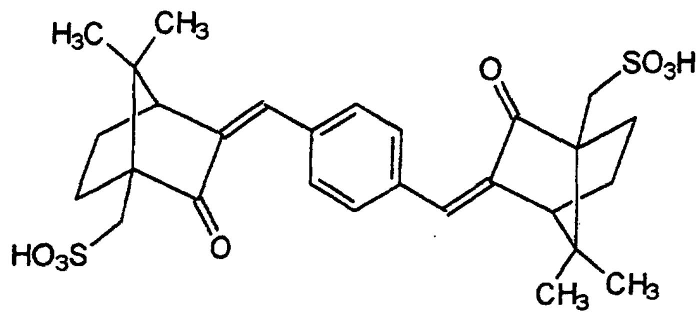

Insbesondere wenn mehrere der unter Normalbedingungen als Festkörper vorliegenden Lichtschutzsubstanzen vorliegen, beispielsweise gewählt aus der Gruppe 4,4',4''-(1,3,5-Triazin-2,4,6-triyltriimino)-tris-benzoesäure-tris (2-ethylhexylester), 4-Methylbenzylidencampher, 4-(tert.-Butyl)-4'-methoxydibenzoylmethan, sind gemäß den Lehren des Standes der Technik nur jeweils geringe Einsatzkonzentrationen und damit niedrige Lichtschutzfaktoren möglich, es sei denn, der Anteil der Ölphase würde überproportional erhöht, was aber ebenfalls Nachteile hätte.

Dennoch bestand der Nachteil des Standes der Technik, daß in der Regel entweder nur vergleichsweise niedrige Lichtschutzfaktoren erreicht werden konnten, oder daß die Lichtschutzfilter nicht die genügende UV-Stabilität aufwiesen oder nicht genügende physiologische Verträglichkeit aufwiesen oder nicht genügend hohe Löslichkeit oder Dispergierbarkeit in kosmetischen oder dermatologischen Zubereitungen aufwiesen oder auch sonstige Inkompatibilitäten mit kosmetischen oder dermatologischen Zubereitungen oder mehrere Nachteile zugleich.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Die anorganischen Pigmente zeichnen sich an sich durch gute Lichtschutzwirkung aus. Sie haben jedoch den Nachteil, daß es schwierig ist, sie in befriedigender Weise solchen Formulierungen einzuverleiben. Nur wenn die Partikel in der endgültigen Formulierung sehr klein sind, werden sie nach dem Auftragen auf die Haut nicht als störendes "Weißeln" (Bildung von weißen Flecken auf der Haut) beobachtet. Üblicherweise liegen die Partikelgrößen solcher Pigmente im Bereich unter 100 nm. In einer herkömmlichen Emulsion neigen die Partikel mehr oder weniger stark zur Zusammenlagerung zu Agglomeraten, welche bereits unter dem Lichtmikroskop zu erkennen sind. Solche Agglomeration ist ferner nicht mit dem Herstellungsprozeß einer entsprechenden Zubereitung abgeschlossen, sondem setzt sich während der Lagerung fort. Das "Weißeln" kann sich daher über einen längeren Zeitraum noch verstärken. Auch kann das Ausölen oder gar Brechen einer Emulsion mittel- oder langfristig Folge einer derartigen Agglomeration sein.

Ein weiterer Nachteil des Einsatzes anorganischer Pigmente in kosmetischen Formulierungen ist, daß solche Pigmente in den weitaus meisten Fällen zu starker Hauttrockenheit führen.

Wenigstens einigen, wenn nicht allen diesen Nachteilen abzuhelfen, war eine der Aufgaben der vorliegenden Erfindung.

Es war indes überraschend und für den Fachmann nicht vorauszusehen, daß

- mit einer oder mehreren im UV-A-Bereich und/oder im UV-B-Bereich absorbierenden organischen Substanzen,

  - wobei eine als Einzelsubstanz vorliegende UV-Filtersubstanz einen Festpunkt oberhalb von 35° C, insbesondere oberhalb von 50° C, aufweist, oder
  - wobei eine Mischung mehrerer UV-Filtersubstanzen einen Festpunkt oberhalb von 35° C, insbesondere oberhalb von 50° C, aufweist, und
  - wobei die Verwendung der UV-Filtersubstanz oder der Mischung mehrerer UV-Filtersubstanzen als kosmetische oder dermatologische Lichtschutzfilter aus kosmetischer oder dermatologischer Sicht unbedenklich ist,

  beschichtete anorganische Pigmente,
  erhältlich dadurch, daß
- ein oder mehrere anorganische Pigmente

  - zu einer Schmelze der im UV-A-Bereich und/oder im UV-B-Bereich absorbierenden organischen Substanz oder
  - zu einer Schmelze der Mischung mehrerer im UV-A-Bereich und/oder im UV-B-Bereich absorbierenden organischen Substanzen gegeben wird,

- die Temperatur des entstandenen Gemisches aus Pigment oder Pigmenten einerseits und UV-Filtersubstanz oder UV-Filtersubstanzen andererseits zumindest soweit abgesenkt wird, daß das Gemisch vollständig als Festkörper vorliegt, vorteilhaft bis auf Raumtemperatur (ca. 20° C),
- und das entstandene Gemisch hernach gewünschtenfalls mit üblichen Verfahren aufbereitet wird,

den Nachteilen des Standes der Technik abhelfen.

Als vorteilhafte Verkörperung der vorliegenden Erfindung wird ebenfalls angesehen ein Verfahren zur Herstellung von

- mit einer oder mehreren im UV-A-Bereich und/oder im UV-B-Bereich absorbierenden organischen Substanzen,
  - wobei eine als Einzelsubstanz vorliegende UV-Filtersubstanz einen Festpunkt oberhalb von 35° C, insbesondere oberhalb von 50° C, aufweist, oder
  - wobei eine Mischung mehrerer UV-Filtersubstanzen einen Festpunkt oberhalb von 35° C, insbesondere oberhalb von 50° C, aufweist, und
  - wobei die Verwendung der UV-Filtersubstanz oder der Mischung mehrerer UV-Filtersubstanzen als kosmetische oder dermatologische Lichtschutzfilter aus kosmetischer oder dermatologischer Sicht unbedenklich ist,

  beschichteten anorganischen Pigmente,
  dadurch gekennzeichnet, daß
- ein oder mehrere anorganische Pigmente
  - zu einer Schmelze der im UV-A-Bereich und/oder im UV-B-Bereich absorbierenden organischen Substanz oder
  - zu einer Schmelze der Mischung mehrerer im UV-A-Bereich und/oder im UV-B-Bereich absorbierenden organischen Substanzen gegeben wird,
- die Temperatur des entstandenen Gemisches aus Pigment oder Pigmenten einerseits und UV-Filtersubstanz oder UV-Filtersubstanzen andererseits zumindest soweit abgesenkt wird, daß das Gemisch vollständig als Festkörper vorliegt, vorteilhaft bis auf Raumtemperatur (ca. 20° C)
- und das entstandene Gemisch hernach gewünschtenfalls mit üblichen Verfahren aufbereitet wird.

Die erfindungsgemäßen, mit UV-Filtersubstanzen beschichteten Pigmente stellen physikalisch und chemisch stabile Addukte dar, welche sich durch vorzügliche Lichtschutzeigenschaften auszeichnen. Es sind Produkte mit erstaunlich erhöhten Lichtschutzfaktoren herstellbar.

Es ist erfindungsgemäß möglich, einem Lichtschutzprodukt zusätzlich zu einem oder mehreren erfindungsgemäßen, mit UV-Filtersubstanzen beschichteten Pigmenten die diesen Addukten zugrundeliegenden UV-Filtersubstanzen zuzugeben und auf diese Weise den Gesamtgehalt an wirksamen UV-Filtersubstanzen zu erhöhen. Dies ist insbesondere deshalb von unverhofftem Vorteil, weil die geringe Löslichkeit einiger UV-Filtersubstanzen der Formulierungskunst bisher Grenzen gesetzt hatte. Durch die Verwendung beispielsweise einer gegebenen UV-Filtersubstanz an der Obergrenze ihrer Löslichkeit und einem erfindungsgemäßen, mit derselben UV-Filtersubstanz beschichteten Pigmente kann der aktive Gesamtgehalt der gegebenen UV-Filtersubstanz jenseits ihrer Löslichkeitsgrenze liegen.

In den erfindungsgemäßen Wirkstoffkombinationen bzw. in kosmetischen oder dermatologischen Zubereitungen, diese Wirkstoffkombinationen enthaltend, haben ferner auch die schwererlöslichen Komponenten eine bessere Löslichkeit als in den Zubereitungen des Standes der Technik, auch dann, wenn mehrere solcher Komponenten vorliegen.

Erfindungsgemäß kann ferner die Agglomeration eventuell vorhandener anorganischer Pigmentpartikel (welche natürlich dispergiert, und nicht gelöst, vorliegen) mit den Folgen "Weißeln", Ausölen, Brechen der Emulsion verhindert werden, auch dann, wenn zusätzlich ein oder mehrere schwererlösliche Komponenten vorliegen.

Weiterhin sind erfindungsgemäß Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Insbesondere die Stabilität von 4-(tert.-Butyl)-4'-methoxydibenzoylmethan wird drastisch erhöht.

Weiterhin sind erfindungsgemäß besonders gut hautverträgliche Zubereitungen erhältlich.

Es ist erfindungsgemäß möglich die Einsatzmengen von 4,4',4"-(1,3,5-Triazin-2,4,6-triyltriimino)-tris-benzoesäure-tris(2-ethylhexylester), aber auch der anderen unter Normalbedingungen als Feststoff vorliegenden Lichschutzfiltersubstanzen, in kosmetischen oder dermatologischen Zubereitungen gegenüber dem Stande der Technik wesentlich zu erhöhen.

Weiterhin war erstaunlich, daß die UV-Stabilität der einzelnen eingesetzten UV-Filtersubstanzen, insbesondere des 4-(tert.-Butyl)-4'-methoxydibenzoylmethans, erfindungsgemäß erheblich erhöht werden kann.

Um zu den erfindungsgemäßen, mit UV-Filtersubstanzen beschichteten Pigmenten zu gelangen, kann vorteilhaft von anorganischen Pigmenten auf Basis von Metalloxiden und/oder anderen in Wasser schwerlöslichen oder unlöslichen Metallverbindungen, ausgegangen werden, insbesondere von Oxiden des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um dabei um Pigmente auf der Basis von $TiO_2$.

Solche anorganischen Pigmente liegen vorteilhaft in hydrophober Form vor, d.h., daß sie oberflächlich wasserabweisend behandelt sind. Diese Oberflächenbehandlung - im Sinne der vorliegenden Erfindung eine Vorbehandlung - kann darin bestehen, daß die Pigmente nach an sich bekannten Verfahren mit einer dünnen hydrophoben Schicht versehen werden.

Ein besonders bevorzugtes Verfahren zur Vorbehandlung anorganischer Pigmente besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter der Handelsbezeichnung T 805 von der Firma Degussa erhältlich.

Es kann auch von Vorteil sein, auf anderem Wege oberflächlich hydrophobierte Pigmente einzusetzen, beispielsweise solche Pigmente, die mit Aluminiumstearat oder ähnlichen Substanzen beschichtet sind.

Vorteilhafte mit Aluminiumstearat beschichtete $TiO_2$-Pigmente sind beispielsweise unter der Handelsbezeichnung M 212 von der Firma Kemira erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 10,0, insbesondere 0,5 - 6,0 Gew.-% gewählt, bezogen auf das Gesamtgewicht der Zubereitungen.

Erfindungsgemäße, mit UV-Filtersubstanzen beschichtete Pigmente sind grundsätzlich vorteilhaft erhältlich ausgehend von allen unter Normalbedingungen als Festkörper vorliegenden UV-Filtersubstanzen, insbesondere von Dibenzoylmethanderivaten, insbesondere von 5-Isopropyldibenzoylmethan und/oder 4-(tert.-Butyl)-4'-methoxydibenzoylmethan, von Benzylidencampherderivaten, insbesondere von Benzylidencampher und/oder 4-Methylbenzylidencampher, von Triazinderivaten, insbesondere vom 4,4',4"-(1,3,5-Triazin-2,4,6-triyltiimino)-tris-benzoësäure-tris (2-ethylhexylester), von Benzophenonderivaten, insbesondere von Benzophenon-1, Benzophenon-2, Benzophenon-3, Benzophenon-4, Benzophenon-6, Benzophenon-8, Benzophenon-9, Benzophenon-11 Benzophenon-12, ferner von 2-cyano-3,3'-diphenylacrylsäurealkylestem, z.B. 2-cyano-3,3'-diphenylacrylsäureethylester, von 2-Phenylbenzimidazol-5-sulfonsäure und ihren Salzen, beispielsweise vom Natrium-, Kalium- oder ihrem Triethanolammonium-Salz, von Sulfonsäure-Derivaten von Benzophenonen, vorzugsweise von 2-Hydroxy-4-methoxybenzophenon-5-sulfonsäure und ihre Salzen, beispielsweise dem entsprechenden Natrium-, Kalium- oder Triethanolammonium-Salz, von Sulfonsäure-Derivaten des 3-Benzylidencamphers, wie z.B. 4-(2-Oxo-3-bornylidenmethyl)benzolsulfonsäure und ihren Salzen, beispielsweise dem entsprechende Natrium-, Kalium- oder Triethanolammonium-Salz:, 2-Methyl-5-(2-oxo-3-bornylidenmethyl)benzolsulfonsäure und ihren Salzen, beispielsweise dem entsprechende Natrium-, Kalium- oder Triethanolammoniun-Salz, sowie von 1,4-di(2-oxo-10-Sulfo-3-bornylidenmethyl)Benzol und dessen Salzen (den entprehenden 10-Sulfato-verbindungen, beispielsweise dem entsprechende Natrium-, Kalium- oder Triethanolammonium-Salz), auch als Benzol-1,4-di(2-oxo-3-bornylidenmethyl-10-Sulfonsäure bezeichnet.

Die Herstellung der erfindungsgemäßen, mit UV-Filtersubstanzen beschichteten Pigmente erfolgt in einfacher Weise dadurch, daß zu einer Schmelze der zugrundeliegenden UV-Filtersubstanz oder einem Gemisch aus zugrundeliegenden UV-Filtersubstanzen ein zugrundeliegendes anorganisches Pigment oder ein Gemisch aus zugrundeliegenden anorganischen Pigmenten gegeben und die entstandene Mischung vermengt wird. Die Mischung wird abgekühlt und kann, ohne weitere obligatorische Aufbereitungsschritte, eingesetzt werden. Es wird allerdings bevorzugt, die abgekühlte Mischung zu mikronisieren, beispielsweise im Kugelmahlverfahren oder über andere dem Fachmanne an sich geläufige Verfahren.

Es ist von Vorteil, bei der Herstellung der erfindungsgemäßen, mit UV-Filtersubstanzen beschichteten Pigmente die Gewichtsverhältnisse von UV-Filtersubstanzen zu anorganischem Pigment aus dem Bereich von 10: 1 bis 1 : 10, bevorzugt 5 : 1 bis 1 : 5 zu wählen, insbesondere ungefähr 1 : 1.

Es ist erfindungsgemäß vorteilhaft, außer den erfindungsgemäßen, mit UV-Filtersubstanzen beschichteten Pigmenten weitere öllösliche UVA-Filter und/oder UVB-Filter in der Lipidphase und/oder weitere wasserlösliche UVA-Filter und/oder UVB-Filter in der wäßrigen Phase einzusetzen, ungeachtet dessen, ob diese unter Normalbedingungen als Festkörper oder als Flüssigkeit vorliegen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen.

Die weiteren UVB-Filter können öllöslich oder wasserlöslich sein. Vorteilhafte öllösliche UVB-Filtersubstanzen sind z.B.:

- weitere 3-Benzylidencampher-Derivate
- 4-Aminobenzoesäure-Derivate, vorzugsweise 4-(Dimethylamino)-benzoesäure(2-ethylhexyl)ester, 4-(Dimethylamino)benzoesäureamylester;
- weitere Ester der Zimtsäure, beispielsweise 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bornylidenmethy))benzolsulfonsäure, 2-Methyl-5-(2-oxo-3-bomylidenmethyl)sulfonsäure und deren Salze.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit UVA-Filtem zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner kann gegebenenfalls von Vorteil sein, die erfindungsgemäßen Wirkstoff-kombinationen mit weiteren UVA- und/oder UVB-Filtern zu kombinieren, beispielsweise bestimmten Salicylsäurederivaten wie

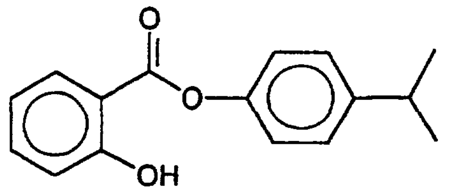

**(4-Isopropylbenzylsalicylat),**

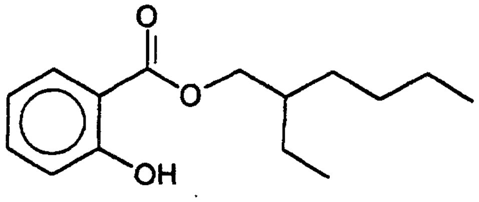

**(2-Ethylhexylsalicylat, Octylsalicylat),**

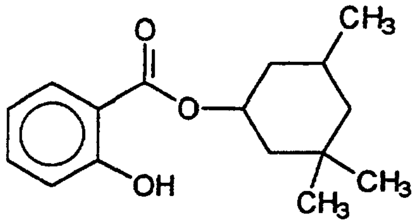

**(Homomenthylsalicylat).**

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 10,0 Gew.-%, bevorzugt 0,5 - 5,0 Gew.-% zu wählen.

Die Gesamtmenge an gegebenenfalls zusätzlichem 4,4',4"-(1,3,5-Triazin-2,4,6-triyltriimino)-tris-benzoesäure-tris(2-ethylhexylester) in den fertigen kosmetischen oder dermatologischen Zubereitungen wird vorteilhaft aus dem Bereich von 0,1-10,0 Gew.-%, bevorzugt 0,5 - 6,0 Gew.-% gewählt, bezogen auf das Gesamtgewicht der Zubereitungen.

Die Gesamtmenge an gegebenenfalls zusätzlichem 4-(tert.-Butyl)-4'-methoxydibenzoylmethan in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an gegebenenfalls zusätzlichem 4-Methylbenzylidencampher in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an gegebenenfalls zusätzlicher 2-Phenylbenzimidazol-5-sulfonsäure bzw. deren Salzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an gegebenenfalls zusätzlichem 2-Ethylhexyl-p-methoxycinnamat in den fertigen kosmetischen oder dermatologischen Zubereitungen wird vorteilhaft aus dem Bereich von 0,1 - 15,0 Gew.-%, bevorzugt 0,5 - 7,5 Gew.-% gewählt, bezogen auf das Gesamtgewicht der Zubereitungen.

Die Gesamtmenge an gegebenenfalls zusätzlichem Ethylhexyl-2-cyano-3,3-diphenylacrylat in den fertigen kosmetischen oder dermatologischen Zubereitungen wird vorteilhaft aus dem Bereich von 0,1 - 15,0 Gew.-%, bevorzugt 0,5 - 10,0 Gew.-% gewählt, bezogen auf das Gesamtgewicht der Zubereitungen.

Es kann auch vorteilhaft sein, den erfindungsgemäßen Zubereitungen zusätzliche hydrophile oder hydrophobierte Pigmente zuzufügen, welche nicht gemäß der Erfindung mit UV-Filtersubstanzen beschichtet sind. Die Gesamtmenge an solchen zusätzlichen, "unbehandelten" anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butylund Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1-10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.
Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.
Von den Kohlenwasserstoffen sind Paraffinöl, Squalan und Squalen vorteilhaft im Sinne der vorliegenden Erfindung zu verwenden.
Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.
Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).
Besonders vorteilhaft sind ferner Mischungen aus Cyclomethicon und Isotridecylisononanoat, aus Cyclomethicon und 2-Ethylhexylisostearat.
Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, - monoethyl- oder -monobutylether, Diethylenglykolmonomethyl- oder - monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Das nachfolgende Beispiel soll die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Herstellungsbeispiele für erfindungsgemäße, mit UV-Filtersubstanzen beschichtete Pigmente

| Verwendete Substanzen: | | Schmelzpunkt |
|---|---|---|
| Silanisiertes $TiO_2$ | T 805, Degussa | |
| mit Al-Stearat behandeltes $TiO_2$ | M 212, Kemira | |
| 4-(tert.-Butyl)-4'-methoxydibenzoylmethan | Parsol 1789, Givaudan | 83,4° |
| 4,4',4''-(1,3,5-Triazin-2,4,6-triyltriimino-) tris-benzoësäure-tris (2-ethylhexylester) | Uvinul T 150, BASF | 125,4° |

Beispiel A

| | Gew.-% |
|---|---|
| M 212 | 85,298 |
| Parsol 1789 | 14,702 |

Das anorganische Pigment wird mit der organischen UV-Filtersubstanz zunächst nur lokker in einer Reibschale oder in einem geschlossenen Rundkolben vermischt. Die Mischung wird anschließend über einen Zeitraum von 30 Minuten in einer Planetenkugelmühle bei 50 UpM trocken vermahlen. Es folgt eine dreistündige thermische Nachbehandlung der Mischung über dem Schmelzpunkt des chemischen Filters im Trockenschrank (140 °C).
Hernach wird das entstandene Produkt desagglomeriert und gegebenenfalls gesiebt.

Beispiel B

| | Gew.-% |
|---|---|
| T 805 | 86,580 |
| Parsol 1789 | 13,420 |

Das anorganische Pigment wird mit der organischen UV-Filtersubstanz zunächt nur lokker in einer Reibschale oder in einem geschlossenen Rundkolben vermischt. Die Mischung wird anschließend über einen Zeitraum von 30 Minuten in einer Planetenkugelmühle bei 50 UpM trocken vermahlen. Es folgt eine dreistündige thermische Nachbehandlung der Mischung über dem Schmelzpunkt des chemischen Filters im Trockenschrank (140 °C).
Hernach wird das entstandene Produkt desagglomeriert und gegebenenfalls gesiebt.

Beispiel C

| | Gew.-% |
|---|---|
| M 212 | 89,735 |
| Uvinul T 150 | 10,265 |

Das anorganische Pigment wird mit der organischen UV-Filtersubstanz zunächt nur lokker in einer Reibschale oder in einem geschlossenen Rundkolben vermischt. Die Mischung wird anschließend über einen Zeitraum von 30 Minuten in einer Planetenkugelmühle bei 50 UpM trocken vermahlen. Es folgt eine dreistündige thermische Nachbehandlung der Mischung über dem Schmelzpunkt des chemischen Filters im Trockenschrank (140 °C).
Hernach wird das entstandene Produkt desagglomeriert und gegebenenfalls gesiebt.

Beispiel D

| | Gew.-% |
|---|---|
| T 805 | 90,672 |
| Uvinul T 150 | 9,328 |

Das anorganische Pigment wird mit der organischen UV-Filtersubstanz zunächt nur lokker in einer Reibschale oder in einem geschlossenen Rundkolben vermischt. Die Mischung wird anschließend über einen Zeitraum von 30 Minuten in einer Planetenkugelmühle bei 50 UpM trocken vermahlen. Es folgt eine dreistündige thermische Nachbehandlung der Mischung über dem Schmelzpunkt des chemischen Filters im Trockenschrank (140 °C).
Hernach wird das entstandene Produkt desagglomeriert und gegebenenfalls gesiebt.

**Beispiel 1**

| O/VV-Lotion | |
|---|---|
| | Gew.-% |
| Glycerylstearat SE | 3,50 |
| Stearinsäure | 1,80 |
| Glycerin | 3,00 |
| Cetearylalkohol | 0,50 |
| Natriumhydroxid (45%ig) | 0,20 |

(fortgesetzt)

| O/VV-Lotion | |
|---|---|
| | Gew.-% |
| Octyldodecanol | 7,00 |
| Caprylyl Ether | 8,00 |
| Uvinul®T150 | 3,00 |
| erfindungsgemäßes Produkt gem. Beispiel A | 4,00 |
| Carbomer | 0,20 |
| Konservierungsmittel | q.s. |
| Parfum | q.s. |
| Wasser, demin. | ad 100,00 |

**Beispiel 2**

| O/VV-Lotion | |
|---|---|
| | Gew.-% |
| Glycerylstearat SE | 3,50 |
| Stearinsäure | 1,80 |
| Glycerin | 3,00 |
| Cetearylalkohol | 0,50 |
| Natriumhydroxid (45%ig) | 0,20 |
| Octyldodecanol | 7,00 |
| Caprylyl Ether | 8,00 |
| Uvinul®T150 | 3,00 |
| erfindungsgemäßes Produkt gem. Beispiel B | 3,00 |
| Octocrylen | 10,00 |
| Eusolex®6300 | 2,00 |
| Carbomer | 0,20 |
| Konservierungsmittel | q.s. |
| Parfum | q.s. |
| Wasser, demin. | ad 100,00 |

**Beispiel 3**

| Hydrodispersionsgel | |
|---|---|
| | Gew.-% |
| Pemulen TR-1 | 0,50 |
| Ethanol | 3,50 |
| Glycerin | 3,00 |
| Dimethicon | 1,50 |
| Natriumhydroxid (45%ig) | 0,55 |
| Octyldodecanol | 0,50 |
| Capric/Caprylic Triglyceride | 5,00 |
| erfindungsgemäßes Produkt gem. Beispiel C | 6,00 |
| Carbomer | 0,20 |
| Konservierungsmittel | q.s. |

(fortgesetzt)

| Hydrodispersionsgel | |
|---|---|
| | Gew.-% |
| Parfum | q.s. |
| Wasser, demin. | ad 100,00 |

**Beispiel 4**

| Hydrodispersionsgel | |
|---|---|
| | Gew.-% |
| Pemulen TR-1 0,50 | |
| Ethanol | 3,50 |
| Glycerin | 3,00 |
| Dimethicon | 1,50 |
| Natriumhydroxid (45%ig) | 0,55 |
| Octyldodecanol | 0,50 |
| Capric/Caprylic Triglyceride | 5,00 |
| erfindungsgemäßes Produkt gem. Beispiel D | 4,00 |
| Eusolex®232 | 1,00 |
| Carbomer | 0,20 |
| Konservierungsmittel | q.s. |
| Parfum | q.s. |
| Wasser, demin. | ad 100,00 |

**Beispiel 5**

| O/VV Creme | |
|---|---|
| | Gew.-% |
| Glycerylstearat SE | 3,50 |
| Stearinsäure | 3,50 |
| Butylenglykol | 5,00 |
| Cetearylalkohol | 3,00 |
| Natriumhydroxid (45%ig) | 0,35 |
| $C_{12}$-$C_{15}$ Alkyl Benzoate | 10,00 |
| Uvinul®T150 | 4,00 |
| erfindungsgemäßes Produkt gem. Beispiel B | 5,00 |
| Benzophenon 3 | 3,00 |
| Carbomer | 0,20 |
| Konservierungsmittel | q.s. |
| Parfum | q.s. |
| Wasser, demin. | ad 100,00 |

**Beispiel 6**

| W/O-Lotion | |
|---|---|
| | Gew.-% |
| Polyglyceryl-2-Polyhydroxystearat | 3,50 |
| Polyglyceryl-3-Diisostearat | 3,50 |
| Butylenglykol | 5,00 |
| Ceresin | 3,00 |
| Natriumhydroxid (45%ig) | 0,35 |
| $C_{12}$-$C_{15}$Alkyl Benzoate | 10,00 |
| Uvinul ®T150 | 4,00 |
| erfindungsgemäßes Produkt gem. Beispiel D | 4,00 |
| Eusolex®6300 | 1,00 |
| $TiO_2$ (T 805) | 3,00 |
| Vaseline | 2,00 |
| Konservierungsmittel | q.s. |
| Parfum | q.s. |
| Wasser, demin. | ad 100,00 |

**Beispiel 7**

| W/O-Creme | |
|---|---|
| | Gew.-% |
| Polyglyceryl-3-Diisostearat | 3,00 |
| Glycerin | 3,00 |
| Polyglyceryl-2-Polyhydroxystearat | 3,00 |
| Kochsalz | 1,00 |
| Isopropylstearat | 7,00 |
| Caprylyl Ether | 5,00 |
| Ceresin | 2,00 |
| erfindungsgemäßes Produkt gem. Beispiel D | 10,00 |
| Parsol® 1789 | 2,00 |
| Isosorbidmonolaurat | 5,00 |
| Konservierungsmittel | q.s. |
| Parfum | q.s. |
| Wasser, demin. | ad 100.0 |

**Patentansprüche**

**1.** Mit einer oder mehreren im UV-A-Bereich und/oder im UV-B-Bereich absorbierenden organischen Substanzen,

- wobei eine als Einzelsubstanz vorliegende UV-Filtersubstanz einen Festpunkt oberhalb von 35° C, insbesondere oberhalb von 50° C, aufweist, oder
- wobei eine Mischung mehrerer UV-Filtersubstanzen einen Festpunkt oberhalb von 35° C, insbesondere oberhalb von 50° C, aufweist, und
- wobei die Verwendung der UV-Filtersubstanz oder der Mischung mehrerer UV-Filtersubstanzen als kosmetische oder dermatologische Lichtschutzfilter aus kosmetischer oder dermatologischer Sicht unbedenklich ist,

beschichtete anorganische Pigmente,
erhältlich dadurch, daß

- ein oder mehrere anorganische Pigmente
  - zu einer Schmelze der im UV-A-Bereich und/oder im UV-B-Bereich absorbierenden organischen Substanz oder
  - zu einer Schmelze der Mischung mehrerer im UV-A-Bereich und/oder im UV-B-Bereich absorbierenden organischen Substanzen gegeben wird,
- die Temperatur des entstandenen Gemisches aus Pigment oder Pigmenten einerseits und UV-Filtersubstanz oder UV-Filtersubstanzen andererseits zumindest soweit abgesenkt wird, daß das Gemisch vollständig als Festkörper vorliegt, vorteilhaft bis auf Raumtemperatur (ca. 20° C),
- und das entstandene Gemisch hernach gewünschtenfalls mit üblichen Verfahren aufbereitet wird.

**2.** Verfahren zur Herstellung von

- mit einer oder mehreren im UV-A-Bereich und/oder im UV-B-Bereich absorbierenden organischen Substanzen,
  - wobei eine als Einzelsubstanz vorliegende UV-Filtersubstanz einen Festpunkt oberhalb von 35° C, insbesondere oberhalb von 50° C, aufweist, oder
  - wobei eine Mischung mehrerer UV-Filtersubstanzen einen Festpunkt oberhalb von 35° C, insbesondere oberhalb von 50° C, aufweist, und
  - wobei die Verwendung der UV-Filtersubstanz oder der Mischung mehrerer UV-Filtersubstanzen als kosmetische oder dermatologische Lichtschutzfitter aus kosmetischer oder dermatologischer Sicht unbedenklich ist,

  beschichteten anorganischen Pigmente,
  **dadurch gekennzeichnet, daß**
- ein oder mehrere anorganische Pigmente
  - zu einer Schmelze der im UV-A-Bereich und/oder im UV-B-Bereich absorbierenden organischen Substanz oder
  - zu einer Schmelze der Mischung mehrerer im UV-A-Bereich und/oder im UV-B-Bereich absorbierenden organischen Substanzen gegeben wird,
- die Temperatur des entstandenen Gemisches aus Pigment oder Pigmenten einerseits und UV-Filtersubstanz oder UV-Filtersubstanzen andererseits zumindest soweit abgesenkt wird, daß das Gemisch vollständig als Festkörper vorliegt, vorteilhaft bis auf Raumtemperatur (ca. 20° C)
- und das entstandene Gemisch hernach gewünschtenfalls mit üblichen Verfahren aufbereitet wird.

**3.** Beschichtete anorganische Pigmente nach Anspruch 1 oder Verfahren zu deren Herstellung nach Anspruch 2, **dadurch gekennzeichnet, daß** sie mit 4-(tert.-Butyl)-4'-methoxydibenzoylmethan beschichtet sind.

## Claims

**1.** Inorganic pigments coated with one or more organic substances absorbing in the UV-A region and/or in the UV-B region,

- where one UV filter substance present as an individual substance has a solidification point above 35°C, in particular above 50°C, or
- where a mixture of two or more UV filter substances has a solidification point above 35°C, in particular above 50°C, and
- where the use of the UV filter substance or the mixture of two or more UV filter substances as cosmetic or

dermatological light protection filters is acceptable from a cosmetic or dermatological point of view,

obtainable by

- adding one or more inorganic pigments
- to a melt of the organic substance absorbing in the UV-A region and/or in the UV-B region or
- to a melt of the mixture of two or more organic substances absorbing in the UV-A region and/or in the UV-B region,
- lowering the temperature of the resulting mixture of pigment or pigments on the one hand and UV filter substance or UV filter substances on the other hand at least sufficiently for the mixture to be completely in the form of a solid, advantageously to room temperature (about 20°C),
- and subsequently processing the resulting mixture if desired using customary processes.

**2.** Process for the preparation of inorganic pigments coated

- with one or more organic substances absorbing in the UV-A region and/or in the UV-B region,
- where one UV filter substance present as an individual substance has a solidification point above 35°C, in particular above 50°C, or
- where a mixture of two or more UV filter substances has a solidification point above 35°C, in particular above 50°C, and
- where the use of the UV filter substance or the mixture of two or more UV filter substances as cosmetic or dermatological light protection filters is acceptable from a cosmetic or dermatological point of view,

**characterized in that**

- one or more inorganic pigments is added
- to a melt of the organic substance absorbing in the UV-A region and/or in the UV-B region or
- to a melt of the mixture of two or more organic substances absorbing in the UV-A region and/or in the UV-B region,
- the temperature of the resulting mixture of pigment or pigments on the one hand and UV filter substance or UV filter substances on the other hand is reduced at least sufficiently for the mixture to be completely in the form of a solid, advantageously to room temperature (about 20°C),
- and the resulting mixture is then worked up if desired using customary processes.

**3.** Coated inorganic pigments according to Claim 1 or process for their preparation according to Claim 2, **characterized in that** they are coated with 4-(tert-butyl)-4'-methoxydibenzoylmethane.

## Revendications

**1.** Pigments minéraux enrobés

- d'une ou plusieurs substances organiques absorbant dans la région UV-A et/ou dans la région UV-B,
- une substance filtrant les UV, présente en tant que substance individuelle, présentant un point de solidification supérieur à 35°C, en particulier supérieur à 50°C, ou
- un mélange de plusieurs substances filtrant les UV présentant un point de solidification supérieur à 35°C, en particulier supérieur à 50°C, et
- l'utilisation de la substance filtrant les UV ou du mélange de plusieurs substances filtrant les UV, en tant que filtre photoprotecteur cosmétique ou dermatologique, étant sans inconvénient du point de vue cosmétique ou dermatologique,

pouvant être obtenus par

- addition d'un ou plusieurs pigments minéraux
  - à une masse fondue de la substance organique absorbant dans la région UV-A et/ou dans la région UV-B ou
  - à une masse fondue du mélange de plusieurs substances organiques absorbant dans la région UV-A et/ou dans la région UV-B,

- abaissement de la température du mélange résultant de pigment ou pigments d'une part et de substance filtrant les UV ou substances filtrant les UV d'autre part, au moins jusqu'à ce que le mélange se trouve en totalité sous forme de solide, avantageusement jusqu'à la température ambiante (environ 20°C),
- et ensuite, si on le désire, traitement final du mélange résultant par des procédés usuels.

**2.** Procédé pour la préparation de pigments minéraux enrobés

- d'une ou plusieurs substances organiques absorbant dans la région UV-A et/ou dans la région UV-B,

    - une substance filtrant les UV, présente en tant que substance individuelle, présentant un point de solidification supérieur à 35°C, en particulier supérieur à 50°C, ou
    - un mélange de plusieurs substances filtrant les UV présentant un point de solidification supérieur à 35°C, en particulier supérieur à 50°C, et
    - l'utilisation de la substance filtrant les UV ou du mélange de plusieurs substances filtrant les UV, en tant que filtre photoprotecteur cosmétique ou dermatologique, étant sans inconvénient du point de vue cosmétique ou dermatologique,

**caractérisé en ce que**

- on ajoute un ou plusieurs pigments minéraux

    - à une masse fondue de la substance organique absorbant dans la région UV-A et/ou dans la région UV-B ou
    - à une masse fondue du mélange de plusieurs substances organiques absorbant dans la région UV-A et/ou dans la région UV-B,

- on abaisse la température du mélange résultant de pigment ou pigments d'une part et de substance filtrant les UV ou substances filtrant les UV d'autre part, au moins jusqu'à ce que le mélange se trouve en totalité sous forme de solide, avantageusement jusqu'à la température ambiante (environ 20°C),
- et ensuite, si on le désire, on soumet à un traitement final le mélange résultant, par des procédés usuels,

**3.** Pigments minéraux enrobés selon la revendication 1 ou procédé pour leur préparation selon la revendication 2, **caractérisés en ce qu'**ils sont enrobés de 4-(tert-butyl)-4'-méthoxydibenzoylméthane.